(19) Europäisches Patentamt
European Patent Office
Office européen des brevets

(11) **EP 4 403 096 A1**

(12) **EUROPEAN PATENT APPLICATION**

(43) Date of publication:
**24.07.2024  Bulletin 2024/30**

(21) Application number: **23305058.2**

(22) Date of filing: **17.01.2023**

(51) International Patent Classification (IPC):
**A61B 5/00** (2006.01)          **A61B 5/024** (2006.01)
**A61B 5/08** (2006.01)          **A61B 5/145** (2006.01)

(52) Cooperative Patent Classification (CPC):
**A61B 5/0022; A61B 5/0205; A61B 5/024;
A61B 5/0816; A61B 5/14542; A61B 5/7267;
A61B 5/7275; A61B 5/746; G16H 40/63;
G16H 40/67; G16H 50/20; G16H 50/30;
G16H 50/50;** G01N 2800/122

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **Biosency
35760 Saint-Grégoire (FR)**

(72) Inventors:
• **TILQUIN, Florian
35510 Cesson Sevigne (FR)**
• **LE LIEPVRE, Sylvain
35510 Cesson Sevigne (FR)**

(74) Representative: **Santarelli
Tour Trinity
1 bis Esplanade de la Défense
92035 Paris La Défense Cedex (FR)**

(54) **METHOD AND SYSTEM FOR MONITORING A REMOTE PATIENT AND TRIGGERING CORRESPONDING ALERTS AND ACTIONS**

(57)     At least one embodiment of a computer-implemented method for monitoring data, the method comprising:
obtaining (200) a high-rate signal representing vital signs of a patient, the vital signs being measured by a plurality of remote sensors worn by the patient,
modelizing (205) the high-rate signal representing the vital signs, the modelized signal representing a behavior of the high-rate signal,
temporally adapting an evolution of the modelized signal, and
triggering (220) an alert as a function of the temporally adapted evolution of the modelized signal.

EP 4 403 096 A1

Fig. 2

## Description

FIELD OF THE DISCLOSURE

The present disclosure relates generally to monitoring remote patients, in particular for early detection of acute exacerbations of chronic obstructive pulmonary disease, to trigger corresponding alerts and actions.

BACKGROUND OF DISCLOSURE

Chronic obstructive pulmonary disease (COPD) is the third leading cause of death worldwide. While COPD is mainly a chronic disease, a substantial number of patients suffer from acute exacerbations. An exacerbation of COPD is a sustained worsening of the patient's condition, from the stable state and beyond normal day-to-day variations that is acute in onset and may warrant additional treatment in a patient with underlying COPD.

Exacerbations of COPD are related to a significantly worse survival outcome. It has been observed that the mortality rate is 21% one year after the first acute exacerbation.

Five main severity classes can be defined for COPD exacerbation:

- mild, that corresponds to an exacerbation treated with antibiotics but no systemic corticosteroid. If no blood gases are available, the absence of respiratory failure is assumed;
- moderate, that corresponds to an exacerbation treated with parenteral corticosteroids with or without antibiotics. If no blood gases are available, the absence of respiratory failure is assumed;
- severe, that is a type 1 respiratory failure with hypoxaemia but no carbon dioxide retention or acidosis; $P_{a,O_2} < 8$ kPa (60 mmHg) and $P_{a,O_2} < 6$ kPa (45 mmHg);
- very severe, that is a type 2 respiratory failure, compensated with hypoxia, carbon dioxide retention but no acidosis; $P_{a,O_2} < 8$ kPa (60 mmHg), $P_{a,O_2} > 6$ kPa (45 mmHg) and hydrogen ion concentration < 44 nM (pH > 7,35); and
- life-threatening, that is a type 2 respiratory failure, decompensated with acidosis and carbon dioxide retention; $P_{a,O_2}$ > 6 kPa (45 mmHg) and hydrogen ion concentration > 44 nM (pH > 7,35).

Severe, very severe, and life-threatening exacerbations result in hospital admissions. According to certain studies, about 140 000 hospital admissions are recorded every year in France and the average length of stay for a patient with an acute exacerbation of COPD is 10 days. The overall cost of hospitalization for acute exacerbation of COPD may be estimated to 1.5 billion € in France every year.

Predicting acute exacerbation of COPD is needed to prevent severe exacerbation by taking care of the patient early enough to avoid severe forms of exacerbations and to reduce patient length of stay at hospital. The early detection of an exacerbation is key to decrease hospital admissions, to improve patient quality of life and to decrease the economic burden associated with COPD exacerbation.

However, no solution for predicting COPD exacerbation is available on the market today. There is therefore a need for COPD patients, for healthcare professionals, and for the healthcare system to develop such a solution to detect COPD exacerbation earlier than the actual care and to trigger corresponding alerts and actions.

SUMMARY OF THE DISCLOSURE

The present disclosure has been devised to address one or more of the foregoing concerns.

According to a first aspect of the disclosure, there is provided a computer-implemented method of monitoring data, the method comprising:

obtaining a high-rate signal representing vital signs of a patient, the vital signs being measured by a plurality of remote sensors worn by the patient,
modelizing the high-rate signal representing the vital signs, the modelized signal representing a behavior of the high-rate signal,
temporally adapting an evolution of the modelized signal, and
triggering an alert as a function of the temporally adapted evolution of the modelized signal.

Accordingly, the method of the disclosure makes it possible to trigger efficiently alerts and/or actions upon detecting cardiopulmonary events or pathologies such as a COPD exacerbation.

According to some embodiments, the method further comprises obtaining medical data of the patient and data of the patient's environment, wherein the alert is triggered as a function of the temporally adapted evolution of the modelized signal, of the obtained medical data of the patient, and/or of the obtained data of the patient's environment.

Still according to some embodiments, the method further comprises computing a score based on a temporally adapted evolution of the modelized signal, the alert being triggered as a function of the computed score and of a threshold.

Still according to some embodiments, the computed score is based on a temporally adapted evolution of the modelized signal, on the obtained medical data of the patient, and on the obtained data of the patient's environment.

Still according to some embodiments, the modelizing comprises a short term modelizing and/or a long-term modelizing.

Still according to some embodiments, the method further comprises normalizing a short term modelized signal using a long-term modelized signal, wherein computing a score comprises analyzing an evolution of the normalized short term modelized data.

Still according to some embodiments, the modelizing comprises a stochastic modelizing, a machine learning regression modelizing, and/or a deep learning modelizing.

The modelizing may comprise a stochastic modelizing comprising a sliding average of the high-rate signal representing the vital signs. It may be carried out independently for each of the vital signs and/or for at least one combination of vital signs.

Still according to some embodiments, the method further comprises filtering the high-rate signal representing the vital signs to remove values that are deemed to be wrong and/or down-sampling the high-rate signal representing vital signs.

Still according to some embodiments, the vital signs comprise oxygen saturation, heart rate, breathing rate, and/or an indicator of the patient's activity.

Still according to some embodiments, the high-rate signal representing the vital signs comprises at least 10 measurements per day, the high-rate signal being automatically obtained without any patient intervention.

Still according to some embodiments, the method further comprises receiving a validation indication in response to triggering an alert, the validation indication being used to strengthen the modelizing the high-rate signal representing the vital signs.

Still according to some embodiments, further comprising receiving a validation indication in response to triggering an alert, wherein the triggering an alert as a function of the temporally adapted evolution of the modelized signal uses a machine learning algorithm, the validation indication being used to strengthen the machine learning algorithm.

According to a second aspect of the disclosure, there is provided a system for early detecting an acute exacerbation of a chronic obstructive pulmonary disease, the system comprising:

> a wearable device comprising:
>
>> a plurality of sensors configured to measure vital signs at a high rate
>> a communication interface to transmit a high rate signal representing the measured vital signs, and
>
> a processing device comprising:
>
>> a communication interface to receive the high rate signal representing the vital signs,
>> a processing unit configured for carrying out each of the steps of the method described above.

This aspect of the disclosure has advantages similar to those mentioned above.

According to other aspects of the disclosure, there is provided a device configured for carrying out each of the steps of the method described above and a non-transitory computer-readable medium storing a program which, when executed by a microprocessor or computer system causes the microprocessor or computer system to perform each step of the method described above.

These aspects of the disclosure have advantages similar to those mentioned above.

At least parts of the methods according to the disclosure may be computer implemented. Accordingly, the present disclosure may take the form of an entirely hardware embodiment, an entirely software embodiment (including firmware, resident software, micro-code, etc.) or an embodiment combining software and hardware aspects that may all generally be referred to herein as a "circuit", "module" or "system". Furthermore, the present disclosure may take the form of a computer program product embodied in any tangible medium of expression having computer usable program code embodied in the medium.

Since the solutions of the present disclosure can be implemented in software, the solutions of the present disclosure can be embodied as computer readable code for provision to a programmable apparatus on any suitable carrier medium. A tangible carrier medium may comprise a storage medium such as a floppy disk, a CD-ROM, a hard disk drive, a magnetic tape device or a solid state memory device and the like. A transient carrier medium may include a signal such as an electrical signal, an electronic signal, an optical signal, an acoustic signal, a magnetic signal or an electromagnetic signal, e.g., a microwave or RF signal.

BRIEF DESCRIPTION OF THE DRAWINGS

Further advantages of the present disclosure will become apparent to those skilled in the art upon examination of the drawings and detailed description. Embodiments of the disclosure will now be described, by way of example only, and with reference to the following drawings, in which:

Figure 1 illustrates schematically an example of an environment wherein some embodiments of the disclosure may be carried out;
Figure 2 illustrates an example of steps of a method for early detecting acute exacerbations of chronic obstructive pulmonary disease and triggering corresponding alerts and actions using an explicit global patient risk score;
Figure 3 illustrates a first example of steps for processing signals representing the vital signs of a patient;
Figure 4 illustrates a second example of steps for processing signals representing the vital signs of a patient;
Figure 5, comprising Figures 5a and 5b, illustrates an example of steps for processing questionnaire data, environmental data, and patient social and clinical data;
Figure 6 illustrates an example of steps for computing risk scores;
Figure 7 illustrates an example of steps for triggering alerts and actions;
Figure 8 illustrates an example of steps of a method for early detecting acute exacerbations of chronic obstructive pulmonary disease and triggering corresponding alerts and actions using an implicit global patient risk score;
Figure 9 is a schematic representation of an example of a wrist-worn portable monitoring device that may be used to acquire data representing vital signs of a patient; and
Figure 10 is a schematic representation of an example of a device configured to implement some embodiments of the disclosure.

DETAILED DESCRIPTION OF THE DISCLOSURE

It is noted that the names of the lists and elements (such as data elements) provided in the following description are only illustrative. The embodiments are not limited thereto, and other names could be used.
According to some embodiments of the disclosure, a signal representing vital signs of a patient is acquired at a high rate by sensors worn by the patient in his/her living environment. This signal is transmitted to servers of a remote monitoring platform, for example a hospital or a medical office, where they are processed with other data such as data relative to the patient and his/her environment, to trigger corresponding alerts and actions, for example to alert a physician and to trigger the arrival of the patient.
For the sake of illustration, the components of the signal to be processed in order to trigger alerts and actions may be obtained through the system known as BORA CARE that has been developed by BIOSENCY (BIOSENCY and BORA CARE are registered trademarks), as described in WO 2019/158704 and WO 2021/064212. BORA CARE comprises a wrist-worn pulse oximeter (BORA Band) collecting the following components that may be transmitted to be displayed and/or processed:

- heart rate;
- breathing rate or respiratory rate;
- oxygen saturation (SpO2);
- number of steps;
- duration of daily activity; and
- skin temperature.

According to some embodiments, the method and system for assisting a healthcare professional in identifying a COPD patient at risk of having an acute exacerbation and for triggering corresponding alerts and actions comprise four main steps:

- collecting components of a signal using sensors worn by the patient, for example the BORA Band that may measure automatically patient heart rate, breathing rate, and oxygen saturation (SpO2) at a high acquisition rate. The patient may wear the connected wristband during 1 to 12 months sessions that can be renewed if needed. During the monitoring session, components of a signal representing vital signs are measured and transmitted to servers in charge of processing the collected data. Still during the monitoring session, other data such as weather data, patient health record and documents, and patient questionnaires are preferably also obtained;
- processing the collected signal components and other data, for example after being rectified (e.g., the sign of SpO2 data is normalized in view of the expected variations of the vital signs). The signal components representing vital signs may be processed to account for short-term variations that can perturb locally the measured signals and may be processed to account for long-term variations that can introduce bias in the value distribution of the vital sign measurements;

- computing risk scores. For example the distribution of vital sign measurements may be compared with short-term and long-term processed data to estimate the risk of having an exacerbation of COPD; and
- triggering alerts and actions, for example by comparing a risk score with a threshold that may be set and updated by a healthcare professional.

It is observed here that computing a risk score may be implicit and thus, an implicit computation of risk scores may be part of triggering alerts and actions. In such a case, the method and system for assisting a healthcare professional in identifying a COPD patient at risk of having an acute exacerbation and for triggering corresponding alerts and actions comprise three main steps.

*System for remotely monitoring a patient for early detection of acute exacerbations of chronic obstructive pulmonary disease and triggering alerts and actions*

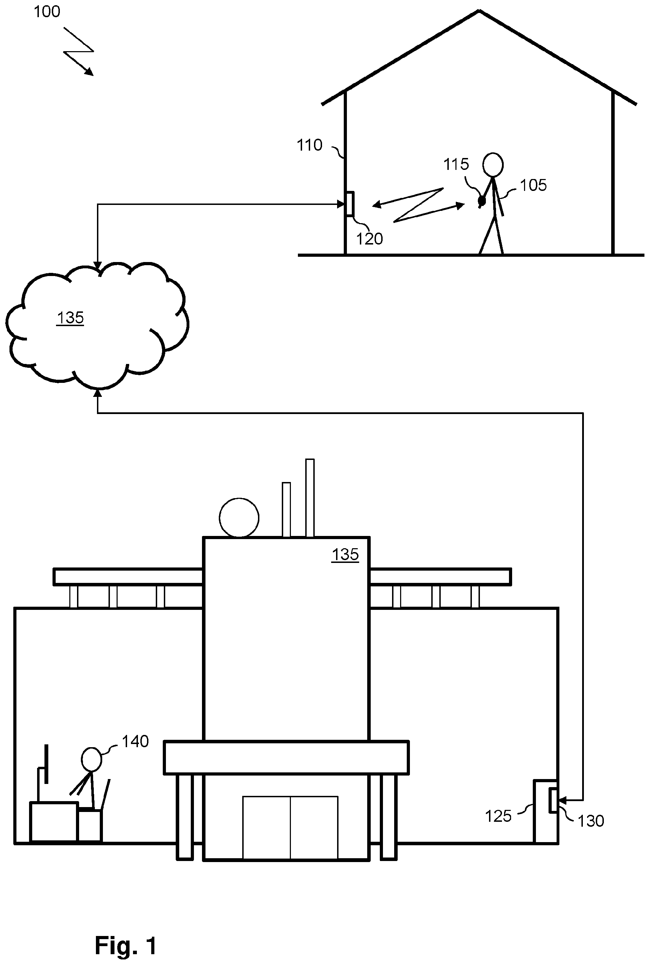

**Figure 1** illustrates schematically an example of an environment wherein some embodiments of the disclosure may be carried out.

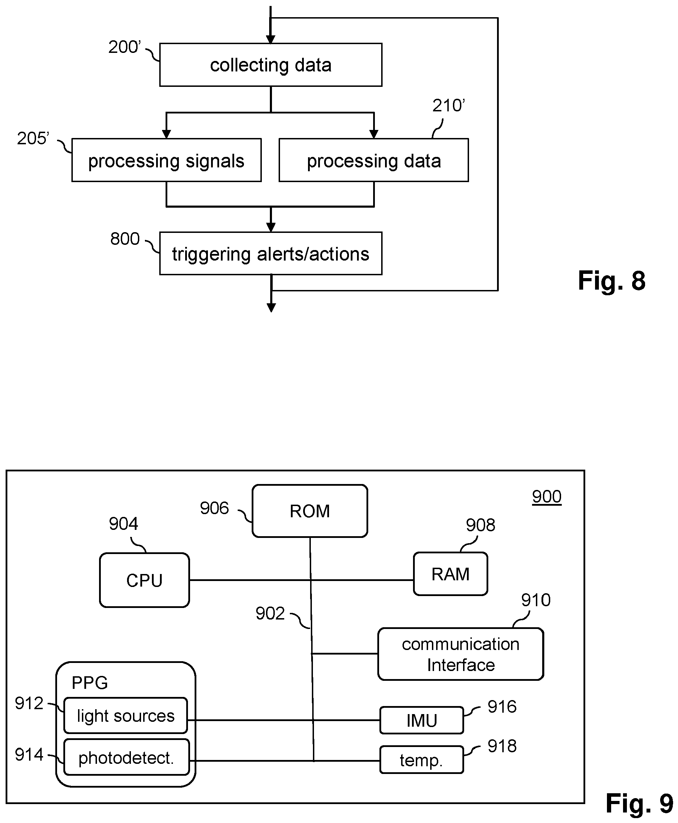

As illustrated, the environment denoted 100 makes it possible to monitor a patient 105 in his/her living environment, for example in his/her house 110, to trigger alerts and actions upon early detection of acute exacerbations of chronic obstructive pulmonary disease. To that end, the patient wears sensors, for example a wrist-worn portable monitoring device 115 such as the BORA Band described above. As illustrated in Figure 9, the wrist-worn portable monitoring device comprises sensors to acquire signal components representing vital signs of the patient and comprises a communication interface to transmit acquired signal components, either raw signal components or processed signal components. These signal components are transmitted to a communication gateway 120, for example using existing communication standards such as Bluetooth (Bluetooth is a registered trademark). From communication gateway 120, the acquired signal components may be transmitted to a server of a remote monitoring platform, for example server 125 comprising a communication interface 130. Transmission of signals from communication gateway 120 to communication interface 130 may be based on any standard communication network 135, for example Internet, using standard communication protocols such as TCP/IP (Transmission Control Protocol / Internet Protocol). Server 125 may be used to process the received data. Alternatively, several servers may be used, for example one server may be used to receive and store the signals and another server may be used to process the received signals. Several servers may also be used to process the received signals.

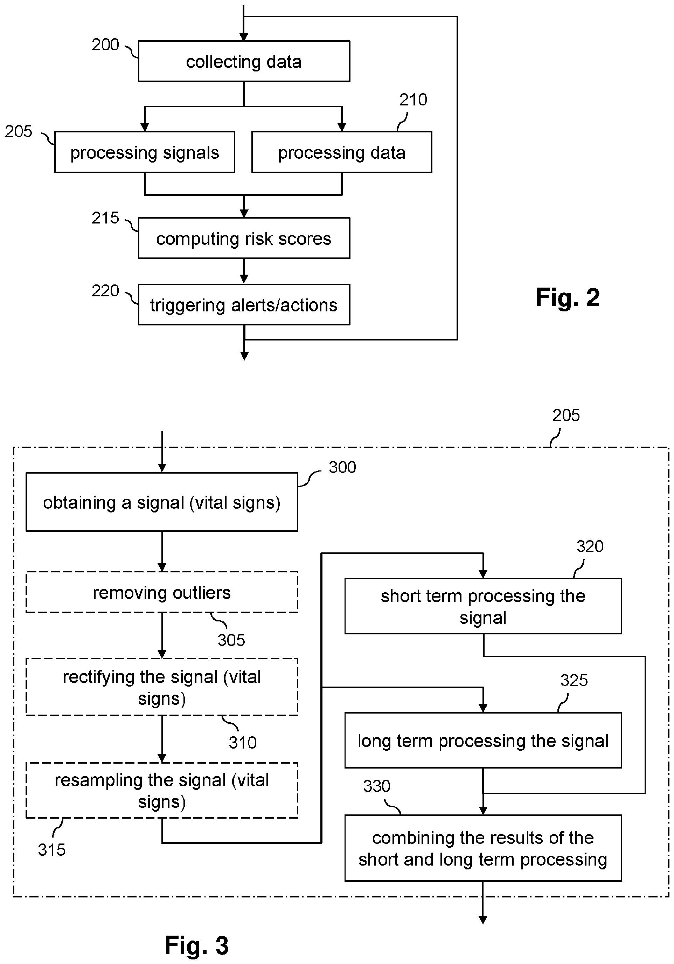

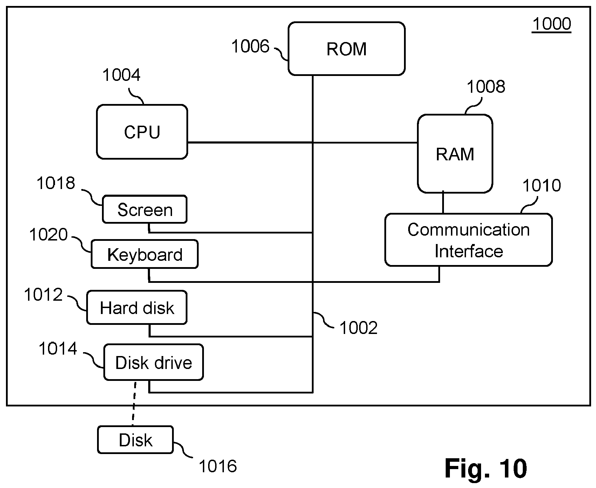

Server 125 may be hosted in a data processing center or close to persons who may access the received signal components, for example in a remote monitoring platform such as a medical office or a hospital, e.g., hospital 135 wherein physician 140 may consult signals and data, set thresholds, etc. Server 125 processes the received signals to trigger alerts and actions, for example to alert physician 140 and to trigger the arrival of patient 105. For the sake of illustration, such actions may comprise calling the patient to investigate the situation, organizing the visit of a nurse at patient's home, ordering an ambulance to pick up the patient at home, booking a hospital room, reserving the necessary equipment, requesting the qualified staff, etc. They may be triggered through dedicated interfaces directed to corresponding software applications. An example of the architecture of server 125 is illustrated in Figure 10. The system illustrated in Figure 1 is only provided as an example. Other configurations exist to implement the disclosure described by reference to Figures 2 to 8.

*Early detecting acute exacerbations of chronic obstructive pulmonary disease and triggering corresponding alerts and actions using an explicit global patient risk score*

**Figure 2** illustrates an example of steps of a method for early detecting acute exacerbations of chronic obstructive pulmonary disease and triggering corresponding alerts and actions.

As illustrated, a first step (step 200) is directed to collecting data. According to some embodiments, four types of data may be obtained: signal components representing vital signs of a patient, regularly updated patient questionnaires, patient's electronic medical records, and/or environmental and social data.

The signal components representing vital signs of the patient, also referred to as the vital sign data collection, may comprise the heart rate, the breathing rate, the oxygen saturation, the skin temperature, the blood pressure, the heart rate variability, the number of steps, the duration of activity, the $CO_2$ saturation, stress metrics, the forced expiratory volume, sleep quality metrics, etc. They may be obtained from a wrist-worn portable monitoring device worn by a patient at home. Such signal components are acquired at a high rate, for example at a frequency defined from one acquisition every ten minutes to one acquisition every two hours. For the sake of illustration, it may be set to one acquisition every twenty minutes.

Patient questionnaires may comprise two types of questionnaires: a patient questionnaire data collection and a caregiver questionnaire data collection. The patient questionnaire data collection may comprise information about symptoms, dyspnea, cough, quality of life, physical activity, work environment, anxiety, fatigue, the presence of family caregivers, etc. They may be entered by the patient using a personal device such as a smartphone or a personal computer provided with an interface making it possible to enter such data and to transmit them to the server in charge of processing the data for early detection of acute exacerbations of chronic obstructive pulmonary disease and for triggering corresponding alerts and actions. The same device may be used for the patient's questionnaire data collection and as the sensor communication box. The caregiver questionnaire data collection may comprise a patient profile, patient observance, the patient ability to use digital devices, etc. They may be entered by a physician or a nurse.

For the sake of illustration, the questionnaires may be updated once a day or less often for the data that may be subject to change (e.g., symptoms and physical activity) and once or twice a year for the others (e.g., patient profile and patient ability to use digital devices).

The patient's electronic medical records, also referred to as patient clinical data collection, correspond to the patient's own data as stored in a patient database within the hospital taking care of the patient. They may comprise the age, gender, BMI (Body Mass Index), smoking status, mMRC (modified Medical Research Council) score, spirometry data, arterial blood gas data, number of exacerbations and hospitalizations during the past 12 months, comorbidities, vaccination status, medicamentation, oxygenotherapy prescription, etc. These data are updated when needed, for example by a physician or a nurse.

The environmental and social data comprise environmental data such as the local weather, air quality index, air concentration of CO, NO, $NO_2$, $O_3$, $SO_2$ gazes, PM2.5 (particle matter smaller than 2.5 microns in size), PM10 (particle matter smaller than 10 microns in size), pollen, and holiday dates that may affect the patient's health. The environmental and social data may also comprise social data such as the social class of the patient, since it has been observed that the patient's social class affects the risk of acute exacerbations of chronic obstructive pulmonary disease. These data may be obtained from standard online databases.

Upon reception, all these signal components and data may be stored locally in the hospital taking care of the patient or remotely, in view of being processed for early detecting acute exacerbations of chronic obstructive pulmonary disease and triggering corresponding alerts and actions.

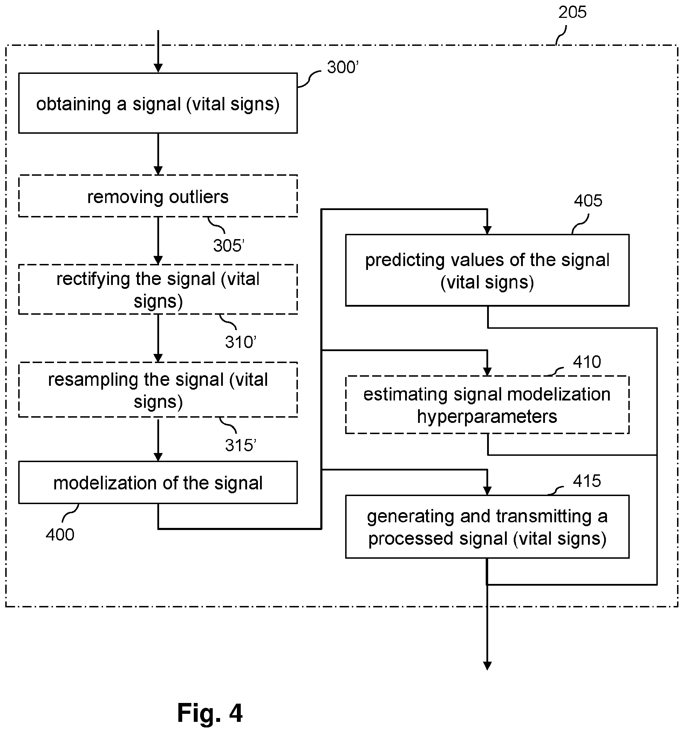

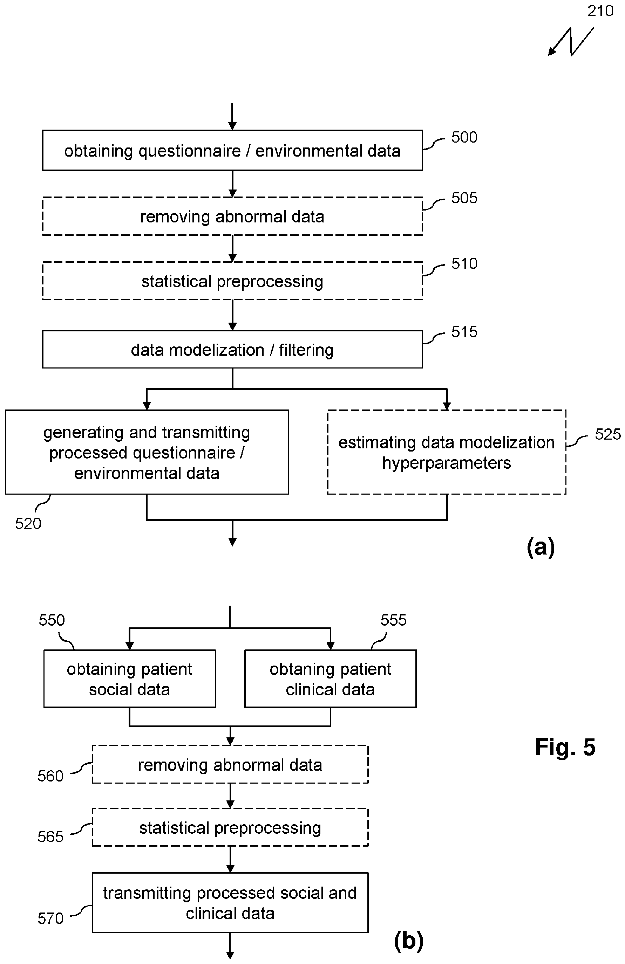

Next, in following steps, the signal components representing the vital signs of the patient and the other data are processed (steps 205 and 210, respectively). A first and a second example of processing the signal components representing the vital signs of the patient are described by reference to Figures 3 and 4, respectively, and an example of processing the other data is described by reference to Figure 5. As described hereafter, processing the signal components representing the vital signs may comprise modelizing the signal representing the vital signs, for example, with one or two models, and temporally adapting evolution of the modelized signal, for example by combining different modelized signals or by combining an actual signal and an expected signal, to highlight some temporal characteristics of the modelized signal.

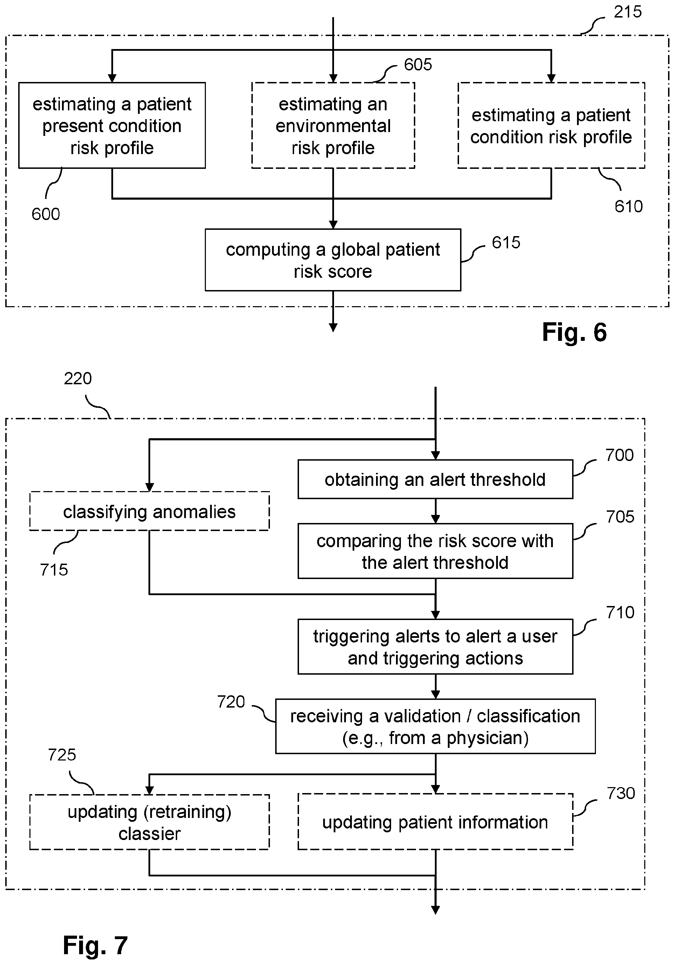

According to the embodiments illustrated in Figure 2, risk scores are computed (step 215) after the signal components representing the vital signs of the patient and the other data are processed. An example of computing risk scores is described by reference to Figure 6.

Next, risk scores are used to trigger alerts and actions (step 220). An example of triggering alerts and actions is described by reference to Figure 7.

*Processing vital sign signals*

**Figure 3** illustrates a first example of steps for processing signals representing the vital signs of a patient.

After having obtained the signal components to be processed (step 300), which may be obtained from a storage server or received from a communication device, the obtained signal components may optionally be preprocessed. Such preprocessing may comprise an analysis to remove outliers (step 305). To that end and for the sake of illustration, simple upper and lower thresholds may be used. Alternatively, the mean (E) and standard deviation (S) of a signal component may be calculated over a sliding window (without considering the value currently analyzed and the previously identified outliers) and the analyzed value is considered an outlier if it does not belong to the range [E - kS; E + kS] where k is a parameter that may be set, for example, to 2.

The preprocessing may also comprise (in addition to the analysis or not) a signal component rectification (step 310), for example a normalization so that the values of the signal component vary in predetermined ranges and/or according to a predetermine trend (or direction). For the sake of illustration, pulsed oxygen saturation (denoted $S_{pO2}$) may be transformed by considering its complementary to 100%, i.e., oxygen desaturation (denoted $D_{O2}$), by the following formula:

$$D_{O2} = 100 - S_{pO2}$$

The preprocessing may also comprise (in addition to the analysis and/or the rectification or not) a resampling (step 315), for example a down-sampling to take into account missing values. For the sake of illustration, the signal components may be acquired at a rate of one value per 20 minutes and resampled so that the signals comprise one value per 30 minutes, for example by computing a median value from values of a signal component present in that 30 minutes window.

Next, a short-term processing may be applied to the signal or to the preprocessed signal if it has been preprocessed (step 320). To that end, the raw signal or the preprocessed signal may be smoothed using a rolling median filter with a predetermined short duration ($d_1$), for example from 6 hours to 24 hours, such as 12 hours. For example, by considering the heart rate (HR), the breathing rate (BR), and the oxygen desaturation ($D_{O2}$), and the time t considered during the follow-up of the patient at the rate of a value every 30 min, the signal may be expressed as follows:

$$X^t = \left( HR^t, BR^t, D_{O_2}^t \right)$$

wherein $\forall t, X^t \in \mathbb{R}^3$

Accordingly, the processed signal may be written as follows:

$$\forall t, X_{ct}^t = median(\{X^{t_i}, t_i \in [t - d_1; t]\})$$

where the median is calculated independently according to each dimension of X (therefore according to each vital sign separately) over a period $d_1$ (e.g., 12 hours) preceding the measurement for which the smoothing value is to be obtained, with the constraint of containing at least *n* measurement points to return a value (none being returned otherwise), *n* being set, for example, to 5. It is to be noted that this median filter does not affect the initial sampling since it is a rolling filter.

In parallel (or before or after), a long-term processing is applied to the signal or to the preprocessed signal if it has been preprocessed (step 325). To that end, the raw signal or the preprocessed signal may be smoothed over a long-time scale in the same way as the smoothing described above with reference to step 320, but over a longer period ($d_2$), for example a period of 15 days. Accordingly, the processed signal may be written as follows:

$$\forall t, X_{lt}^t = median(\{X^{t_i}, t_i \in [t - d_2; t]\})$$

Next, the results from the short term and long-term processing are combined (step 330). According to a particular embodiment, the processed long-term signal component obtained in step 325 are subtracted from the smoothed short term signal components obtained in step 320 in order to constitute a centered signal that may be expressed as follows:

$$\forall t, \widetilde{X^t} = X_{ct}^t - X_{lt}^t$$

This processed signal is transmitted to be used for early detecting acute exacerbations of chronic obstructive pulmonary disease and triggering corresponding alerts and actions. Alternatively, it may be stored to be used later.

It is observed that other short term and/or long-term processing may be applied to the signal or the preprocessed signal. For the sake of illustration, the data smoothing may be based on an exponential moving average and/or on autoregressive models. It is also noted that the short-term processing may be based on a different method than the long-term processing.

**Figure 4** illustrates a second example of steps for processing signals representing the vital signs of a patient. After having obtained the signal components to be processed (step 300'), which may be obtained from a storage server or received from a communication device, the obtained signal components may optionally be preprocessed. Such preprocessing may be similar to those described by reference to steps 305 to 315 in Figure 3. Accordingly, such preprocessing may comprise an analysis to remove outliers (step 305'), a rectification of signal components (step 310'), and/or a resampling (step 315').

Next, the signal is modelized or the model is updated (step 400), based on all (or many of) the signal component values previously obtained. Such a model may be a deterministic model, a stochastic model (e.g., a Kalman filter or a particle filter), a gaussian process regression for each vital sign independently, in which a part of the covariance matrix designed to model the daily evolution of the component values has been removed (for instance a covariance matrix designed to model the daily evolution of the vital sign with an exponential sine squared kernel of length scale equal to one day, the rest of its evolution with a radial basis function (RBF) kernel and the noise with a gaussian noise kernel, in which the circadian evolution is suppressed afterwards (i.e. only the RBF and noise kernels are kept once hyperparameters have been obtained by log-marginal likelihood optimization), in order to observe only the evolution of the heart rate that is different from the natural daily cycle of the patient), a machine learning regression model, and/or a deep learning regression model.

Next, component values are predicted based on the modelized signal (step 405), for example by extrapolation, and signal modelization hyperparameters may optionally be estimated as a function of the model, of the amount of the signal component values previously obtained, of an error estimation, etc. (step 410), to control the modelization training process. For the sake of illustration, with gaussian process regression, hyperparameters of the covariance matrix may be obtained via optimization of the log-marginal likelihood.

Next, a processed signal is generated, based on the raw signal or the preprocessed signal and the predicted values of the signal, and transmitted, to be used for early detecting acute exacerbations of chronic obstructive pulmonary disease and triggering corresponding alerts and actions (step 415). Alternatively, it is stored to be used later. For the sake of illustration, the processed signal may correspond to the difference between the raw signal (or the preprocessed signal) and the predicted signal.

*Processing questionnaire data, environmental data, patient social data, and patient clinical data*

**Figure 5a** illustrates an example of steps for processing questionnaire data or environmental data. As illustrated, a first step is directed to obtaining the questionnaire data or the environmental data (step 500), for example from a storage server or from a communication device. As described above, the questionnaire data may comprise a patient questionnaire data collection and a caregiver questionnaire data collection.

The questionnaire data or the environmental data may optionally be preprocessed. Such preprocessing may comprise an analysis to remove abnormal data (step 505), for example based on an upper and a lower thresholds, or based on data that are deemed meaningless (e.g. a data value that is abnormally constant over time). The preprocessing may also comprise (in addition to the analysis or not) a statistical preprocessing (step 510) that may consist, for example in computing the trend (e.g., a variation from one questionnaire to the previous one or from environmental data obtained at a given time to environmental data obtained at the previous given time) of the standardized sum of the questionnaire results (or of the environmental data) with respect to time.

Next, the data are modelized or the model is updated (step 515), based on all (or many of) the data values previously obtained. For the sake of illustration, this model may be a deterministic model, a stochastic model (e.g., a Kalman filter or a particle filter), a machine learning regression model (e.g., a gaussian process regression), and/or a deep learning regression model. Alternatively, the data may be filtered.

Next, processed questionnaire data or processed environmental data are generated and transmitted to be used for early detecting acute exacerbations of chronic obstructive pulmonary disease and triggering corresponding alerts and actions (step 520). Alternatively, they are stored to be used later. For the sake of illustration, the processed questionnaire data may correspond to the difference between the raw questionnaire data (or the preprocessed questionnaire data) and questionnaire data predicted from the questionnaire data model. Likewise, the processed environmental data may correspond to the difference between the raw environmental data (or the preprocessed environmental data) and environmental data predicted from the environmental data model.

In parallel (or before or after), questionnaire data modelization hyperparameters (or environmental data modelization hyperparameters) may optionally be estimated as a function of the model, of the amount of the questionnaire data (or environmental data) previously obtained, of an error estimation, etc. (step 525), to control the modelization training process.

**Figure 5b** illustrates an example of steps for processing patient social data and patient clinical data. As illustrated, first steps are directed to obtaining the patient social data (step 550) and the patient clinical data (step 555), for example from a storage server or from a communication device.

The patient social and clinical data may optionally be preprocessed. Again, such preprocessing may comprise an analysis to remove abnormal data (step 560), for example based on an upper and a lower thresholds. The preprocessing may also comprise (in addition to the analysis or not) a statistical preprocessing (step 565) that may consist, for example, in renormalizing the data.

Next, processed patient social and clinical data corresponding to the raw patient social and clinical data or to the preprocessed patient social and clinical data are transmitted to be used for early detecting acute exacerbations of chronic obstructive pulmonary disease and triggering corresponding alerts and actions (step 570). Alternatively, they are stored to be used later.

*Computing risk scores*

**Figure 6** illustrates an example of steps for computing risk scores.

As illustrated and according to particular embodiments, a global patient risk score may be computed based on a patient's present condition risk profile and optionally on an environmental risk profile and/or a patient condition risk profile.

For the sake of illustration, the patient present condition risk profile may be estimated (step 600) based on the processed signal (e.g., as described by reference to Figure 3 or 4) and optionally on the processed questionnaire data (e.g., as described by reference to Figure 5a). In addition, estimating the patient present condition risk profile may also be based on the estimated signal modelization hyperparameters and/or on the estimated questionnaire data modelization hyperparameters, when appropriate. Still for the sake of illustration, estimating the patient present condition risk profile may be based on a method such as the z-score, interquartile range, local outlier factor (LOF), anomaly detection algorithm / Outlier detection algorithm (Angle-Based Techniques (ABOD), Depth-Based Techniques (Convex Hull), isolation forest, one-class SVM, autoencoder, In-degree Number, etc.

For example, and considering a signal processed according to the example given by reference to Figure 3, the centered signal (as described by reference to step 330) may be standardized by renormalizing it using the standard deviation of the centered data values reported over the long-term time range $d_2$ (e.g., 15 days). The calculation of the standard deviation may be done independently according to each vital sign:

$$\forall t, \sigma_{lt}\left(\widetilde{X^t}\right) = std\left(\left\{\widetilde{X^{t_i}}, t_i \in [t - d_2; t]\right\}\right)$$

By normalizing the centered signal $\widetilde{X^t}$ -independently according to each vital sign-using the standard deviation (std) thus obtained, a quantity called z-score, denoted $Z^t$, may be obtained as follows:

$$\forall t, Z^t = \frac{\widetilde{X^t}}{\sigma_{lt}\left(\widetilde{X^t}\right)}$$

being noted that the Z-score may be expressed as follows:

$$Z^t = \left(Z^t_{HR}, Z^t_{BR}, Z^t_{DO_2}\right)$$

The Z-score may then be constrained between two values, for example between 0 and 6, and may be smoothed using a rolling average filter like (as described by reference to step 320) over a very short-term duration $d_3$, for example a short-term duration of 3 hours, and by applying a short-term rolling maximum filter (still independently according to each vital sign):

$$\overline{Z^t} = mean(\{Z^{t_i}, t_i \in [t - d_3; t]\})$$

$$\overline{Z^t_{max}} = max\left(\left\{\overline{Z^{t_i}}, t_i \in [t - d_1; t]\right\}\right)$$

For the sake of illustration and still considering only the heart rate (HR), the breathing rhythm (BR), and the oxygen desaturation ($D_{O2}$), the patient present condition risk profile, denoted EXA, may be computed as follows:

$$\forall t, EXA^t = -log\left(S\left(\frac{Z^t_{max,HR} + Z^t_{max,BR} + Z^t_{max,DO_2}}{3}\right)\right)$$

where S is a function that may be defined as follows:

$$S: t \rightarrow \frac{1}{2}\left[1 - erf\left(\frac{x}{\sqrt{2}}\right)\right]$$

and erf is the error function that may be defined as follows:

$$erf: z \rightarrow \frac{2}{\sqrt{\pi}} \int_0^z e^{-t^2} dt$$

Turning back to Figure 6, the environmental risk profile may optionally be estimated (step 605) based on the processed environmental data and a patient population database comprising pathological observations. Estimating an environmental risk profile may be done using a standard machine learning classification algorithm to associate environmental data with pathological observations.

Similarly, the patient condition risk profile may optionally be estimated (step 610) based on the processed social and clinical data and a patient population database comprising pathological observations. Again, estimating a patient risk profile may use a standard machine learning classification algorithm to associate social and clinical data with pathological observations, or it may be computed similarly to the questionnaire data processing.

The estimated patient present condition risk profile, environmental risk profile, and/or patient condition risk profile may be used to compute the global patient risk score (step 615). Such a computation may be done using a logistic regression, a machine learning method, a Bayesian method, or a probability fusion method.

For the sake of illustration and still considering only the patient present condition risk profile (EXA), the environmental risk profile (ER), and/or the patient condition risk profile (PCR), the global patient risk score, denoted GPR, may be computed as follows:

$$GPR = EXA + k \times ER + l \times PCR$$

where k and I represent weight parameters and where the environmental risk and the patient condition risk are optional.

*Triggering alarms and actions*

**Figure 7** illustrates an example of steps for triggering alerts and actions.

As illustrated, a first step is directed to obtaining an alert threshold (step 700). For the sake of illustration, such an alert threshold may be estimated automatically, for example using a machine-learning system, or may be set by a professional, for example a physician. Next, the global patient risk score is compared to the obtained alert threshold (step 705). As a result of the comparison, one or more alerts and one or more actions may be triggered (step 710). For the sake of illustration, triggering an alert may comprise transmitting an alert onto a user interface used, for example, to a physician. The alert may be a visual alert, an audio alert, a vibration alert, etc., or a combination thereof. As described above, the triggered actions may comprise calling the patient to investigate the situation, organizing the visit of a nurse at patient's home, ordering an ambulance to pick up the patient at home, booking a hospital room, reserving the necessary equipment, requesting the qualified staff, etc.

In parallel, a step may be carried out to detect and preferably classify a possible anomaly (step 715). A level of risk may also be provided with the category or categories of the possible anomaly detected.

Such a classification may be based on the computed global patient risk score and optionally, the patient population database, the predicted signal, and/or the predicted questionnaire data, using a supervised classification method (e.g., machine learning or deep learning classification method) that associates the computed global patient risk score and optionally, the patient population database, the predicted signal, and/or the predicted questionnaire data, with one or more categories of anomaly. Such classification may correspond to a class defining a COPD exacerbation. Other classes may correspond to other cardiopulmonary events or pathologies, such as for example sleep apnea, atrial fibrillation, idiopathic pulmonary fibrosis acute exacerbation, asthma exacerbation.

The anomaly classification may lead to triggering different actions, in particular for the physician.

Next, confirmation and/or classification may be received to validate the alerts triggered by comparing the global patient risk score with the threshold and/or the possible anomalies detected (step 720). Such information may be entered by a physician in view of the computed or estimated values, of the circumstances, and of his/her observations.

The confirmation and/or classification received may be used for training purpose, to strengthen learning of the supervised classification algorithm (step 725).

In addition, patient information may be updated based on the confirmation and/or classification received (step 730).

For the sake of illustration and still considering only the patient present condition risk profile (EXA), the environmental risk profile (ER), and the patient condition risk profile (PCR), the Global Patient Risk score, denoted GPR, may be trained through the optimization of the performance (for example maximization of the Area Under Curve (AUC) of the Receiver Operating Curve (ROC) curve, or for example maximization of True Positive Rate (TPR) at constant False Positive Rate (FPR) FPR=5%) of GPR in detecting anomalies labeled in the training database.

*Early detecting acute exacerbations of chronic obstructive pulmonary disease and triggering corresponding alerts and actions using an implicit global patient risk score*

**Figure 8** illustrates an example of steps of a method for early detecting acute exacerbations of chronic obstructive pulmonary disease and triggering corresponding alerts and actions using an implicit global patient risk score.

As illustrated, a first step (step 200') is directed to collecting data, in particular signal components representing the vital signs of the patient. Such a step may be similar to step 200 described in reference to Figure 2.

Next, in following steps, the signal components representing the vital signs of the patient and the other data collected (if any) are processed (steps 205' and 210', respectively). Again, these steps may be similar to steps 205 and 210 described in reference to Figure 2.

Next, the processed signals and the processed data (if any) are used to trigger alerts and actions (step 800). For the sake of illustration, step 800 may comprise steps similar to steps 710 to 730, as described with reference to Figure 7, wherein no global patient risk score is computed but wherein detection and preferably classification are based on the processed signals and the processed data (if any) .

Therefore, the classification and possibly an associated level of risk may be based on the processed signals and the processed data (if any) and optionally, on other data such as a patient population database, using a supervised classification algorithm (e.g., machine learning or deep learning classification method) that associates the processed signals and the processed data (if any) with one or more categories of anomaly. Again, such classification may correspond to a class defining a COPD exacerbation and/or to other classes that may correspond to other cardiopulmonary events or pathologies, such as for example sleep apnea, atrial fibrillation, idiopathic pulmonary fibrosis acute exacerbation, asthma exacerbation.

The anomaly classification may lead to triggering different actions, in particular for the physician.

Similarly to step 720 described with reference to Figure 7, confirmation and/or classification may be received to validate the alerts triggered by the possible anomalies detected. Again, such information may be entered by a physician in view of the computed or estimated values, of the circumstances, and of his/her observations.

The confirmation and/or classification received may be used for training purpose, to strengthen learning of the supervised classification algorithm. In addition, patient information may be updated based on the confirmation and/or classification received.

According to the example illustrated in Figure 8, no global patient risk score is directly accessible, but it is implicitly used by the supervised classification algorithm used to classify a possible anomaly. Depending on the supervised classification algorithm, data representing a global patient risk score may be obtained and provided to a user, for example a physician.

*Example of a hardware to carry out steps of the method of embodiments of the present disclosure*

**Figure 9** is a schematic representation of an example of a wrist-worn portable monitoring device that may be used to acquire data representing vital signs of a patient.

According to particular embodiments, the wrist-worn portable monitoring device 900 comprises a communication bus 902 to which there are preferably connected:

- a central processing unit 904, such as a microprocessor, denoted CPU;
- a read-only memory 906, denoted ROM, for storing computer programs for partially implementing the disclosure;
- a random access memory 908, denoted RAM, for storing the executable code of methods enabling the wrist-worn portable monitoring device to acquire and transmit signals representing vital signs as well as the registers adapted to record variables and parameters necessary for implementing such methods;
- at least one communication interface 910 for transmitting acquired signals; and
- sensors, for example, a photoplethysmogram (PPG) sensor comprising at least two light sources 912 and a photodetector 914 such as a photodiode or a phototransistor, an inertial motion unit 916, and a temperature sensor 918.

Preferably the communication bus provides communication and interoperability between the various elements included in the communication device 900 or connected to it. The representation of the bus is not limiting and in particular the central processing unit is operable to communicate instructions to any element of the communication device 900 directly or by means of another element of the communication device 900.

The executable code may be stored in read-only memory 906.

The central processing unit 904 is preferably adapted to control and direct the execution of the instructions or portions of software code of the program or programs. On powering up, the program or programs that are stored in a non-volatile memory are transferred into the random access memory 908, which then contains the executable code of the program or programs, as well as registers for storing the variables and parameters necessary. Alternatively, the program or programs may be implemented in hardware (for example, in the form of an Application Specific Integrated Circuit or ASIC).

**Figure 10** is a schematic representation of an example of a device configured to implement some embodiments of the present disclosure, such a server 125 in Figure 1.

The device 1000 may preferably be a device such as a micro-computer, a workstation or a light portable device. As illustrated, device 1000 comprises a communication bus 1002 to which there are preferably connected:

- a central processing unit 1004, such as a microprocessor, denoted CPU;
- a read-only memory 1006, denoted ROM, for storing computer programs for implementing (at least partially) the disclosure;
- a random access memory 1008, denoted RAM, for storing the executable code of methods according to embodiments of the disclosure as well as the registers adapted to record variables and parameters necessary for implementing methods according to embodiments of the disclosure; and
- at least one communication interface 1010 to receive signals and data, in particular signals representing vital signs received from a wrist-worn portable monitoring device.

Optionally, communication device 1000 may also include the following components:

- a data storage means 1012 such as a hard disk, for storing computer programs for implementing methods according to one or more embodiments of the disclosure;
- a disk drive 1014 for a disk 1016, the disk drive being adapted to read data from the disk 1016 or to write data onto said disk;
- a screen 1018 for serving as a graphical interface with the user, by means of a keyboard 1020 or any other pointing means.

The communication device 1000 may be optionally connected to various peripherals.

Preferably the communication bus provides communication and interoperability between the various elements included in the communication device 1000 or connected to it. The representation of the bus is not limiting and in particular the central processing unit is operable to communicate instructions to any element of the communication device 1000 directly or by means of another element of the communication device 1000.

The disk 1016 may optionally be replaced by any information medium such as for example a compact disk (CD-ROM), rewritable or not, a ZIP disk, a USB key or a memory card and, in general terms, by an information storage means that can be read by a microcomputer or by a microprocessor, integrated or not into the apparatus, possibly removable and adapted to store one or more programs whose execution enables a method according to the disclosure to be implemented.

The executable code may optionally be stored either in read-only memory 1006, on the hard disk 1012, or on a removable digital medium such as for example a disk 1016 as described previously. According to an optional variant, the executable code of the programs can be received by means of the communication network, via the interface 1010, in order to be stored in one of the storage means of the communication device 1000, such as the hard disk 1012, before being executed.

The central processing unit 1004 is preferably adapted to control and direct the execution of the instructions or portions of software code of the program or programs according to the disclosure, which instructions are stored in one of the aforementioned storage means. On powering up, the program or programs that are stored in a non-volatile memory, for example on the hard disk 1012 or in the read-only memory 1007, are transferred into the random access memory 1012, which then contains the executable code of the program or programs, as well as registers for storing the variables and parameters necessary for implementing the disclosure.

In a preferred embodiment, the apparatus is a programmable apparatus which uses software to implement the disclosure. However, alternatively, the disclosure may be implemented in hardware (for example, in the form of an Application Specific Integrated Circuit or ASIC).

Although the present disclosure has been described herein above with reference to specific embodiments, the disclosure is not limited to the specific embodiments, and modifications will be apparent to a skilled person in the art which lie within the scope of the disclosure.

Many further modifications and variations will suggest themselves to those versed in the art upon making reference to the foregoing illustrative embodiments, which are given by way of example only and which are not intended to limit the scope of the disclosure, that being determined solely by the appended claims. In particular, the different features from different embodiments may be interchanged, where appropriate.

In particular, although the invention has been described mainly in the context of early detection of acute exacerbations of chronic obstructive pulmonary disease, it may be used in other contexts, for example for detecting other types of pathologies, such as the detection of exacerbations of pulmonary fibrosis or the detection of exacerbations in the context of pulmonary tuberculosis.

Each of the embodiments of the disclosure described above can be implemented solely or as a combination of a plurality of the embodiments. Also, features from different embodiments can be combined where necessary or where the combination of elements or features from individual embodiments in a single embodiment is beneficial.

In the claims, the word "comprising" does not exclude other elements or steps, and the indefinite article "a" or "an" does not exclude a plurality. The mere fact that different features are recited in mutually different dependent claims does not indicate that a combination of these features cannot be advantageously used.

**Claims**

1. A computer-implemented method for monitoring data, the method comprising:

   obtaining (200, 300, 300') a high-rate signal representing vital signs of a patient, the vital signs being measured by a plurality of remote sensors worn by the patient,
   modelizing (205, 320, 325, 400) the high-rate signal representing the vital signs, the modelized signal representing a behavior of the high-rate signal,
   temporally adapting (330, 415) an evolution of the modelized signal, and
   triggering (220) an alert as a function of the temporally adapted evolution of the modelized signal.

2. The method of claim 1, further comprising obtaining medical data of the patient and data of the patient's environment, wherein the alert is triggered as a function of the temporally adapted evolution of the modelized signal, of the obtained medical data of the patient, and/or of the obtained data of the patient's environment.

3. The method of claim 1 or claim 2, further comprising computing a score (215) based on a temporally adapted evolution of the modelized signal, the alert being triggered as a function of the computed score and of a threshold.

4. The method of claim 3 depending on claim 2, wherein the computed score is based on a temporally adapted evolution of the modelized signal, on the obtained medical data of the patient, and on the obtained data of the patient's environment.

5. The method of any one of claims 1 to 4, wherein the modelizing comprises a short term modelizing (320) and/or a long-term modelizing (325).

6. The method of claim 5, the method further comprising normalizing a short term modelized signal using a long-term modelized signal, wherein computing a score comprises analyzing an evolution of the normalized short term modelized data.

7. The method of any one of claims 1 to 6, wherein the modelizing (400) comprises a stochastic modelizing, a machine learning regression modelizing, and/or a deep learning modelizing.

8. The method of any one of claims 1 to 7, further comprising filtering (305, 305') the high-rate signal representing the vital signs to remove values that are deemed to be wrong and/or down-sampling (315, 315') the high-rate signal representing vital signs.

9. The method of any one of claims 1 to 8, wherein the vital signs comprise oxygen saturation, heart rate, breathing rate, and/or an indicator of the patient's activity.

10. The method of any one of claims 1 to 9, wherein the high-rate signal representing the vital signs comprises at least 10 measurements per day, the high-rate signal being automatically obtained without any patient intervention.

**11.** The method of any one of claims 1 to 10, further comprising receiving a validation indication in response to triggering an alert, the validation indication being used to strengthen the modelizing the high-rate signal representing the vital signs.

**12.** The method of any one of claims 1 to 10, further comprising receiving a validation indication in response to triggering an alert, wherein the triggering an alert as a function of the temporally adapted evolution of the modelized signal uses a machine learning algorithm, the validation indication being used to strengthen the machine learning algorithm.

**13.** A computer program product for a programmable apparatus, the computer program product comprising a sequence of instructions for implementing each of the steps of the method according to any one of claims 1 to 12 when loaded into and executed by the programmable apparatus.

**14.** A monitoring device comprising a processing unit configured for carrying out each of the steps of the method according to any one of claims 1 to 12.

**15.** A system for early detecting an acute exacerbation of a chronic obstructive pulmonary disease, the system comprising:

a wearable device comprising:

a plurality of sensors configured to measure vital signs at a high rate
a communication interface to transmit a high rate signal representing the measured vital signs, and

a processing device comprising:

a communication interface to receive the high rate signal representing the vital signs,
a processing unit configured for carrying out each of the steps of the method according to any one of claims 1 to 12.

**Fig. 1**

200 collecting data

205 processing signals

210 processing data

215 computing risk scores

220 triggering alerts/actions

**Fig. 2**

205

300 obtaining a signal (vital signs)

removing outliers
305

rectifying the signal (vital signs)
310

resampling the signal (vital signs)
315

320 short term processing the signal

325 long term processing the signal

330 combining the results of the short and long term processing

**Fig. 3**

205

obtaining a signal (vital signs) — 300'

removing outliers — 305'

rectifying the signal (vital signs) — 310'

resampling the signal (vital signs) — 315'

modelization of the signal — 400

predicting values of the signal (vital signs) — 405

estimating signal modelization hyperparameters — 410

generating and transmitting a processed signal (vital signs) — 415

**Fig. 4**

210

obtaining questionnaire / environmental data — 500

removing abnormal data — 505

statistical preprocessing — 510

data modelization / filtering — 515

generating and transmitting processed questionnaire / environmental data

estimating data modelization hyperparameters — 525

520

**(a)**

550

555

obtaining patient social data

obtaning patient clinical data

560 — removing abnormal data

565 — statistical preprocessing

570 — transmitting processed social and clinical data

**(b)**

**Fig. 5**

**Fig. 6**

**Fig. 7**

200' collecting data

205' processing signals          210' processing data

800 triggering alerts/actions

**Fig. 8**

900

906 ROM

904 CPU          908 RAM

902

910 communication Interface

PPG
912 light sources          916 IMU
914 photodetect.          918 temp.

**Fig. 9**

Fig. 10

Europäisches
Patentamt

European
Patent Office

Office européen
des brevets

## EUROPEAN SEARCH REPORT

**Application Number**

EP 23 30 5058

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2020/297955 A1 (SHOULDICE REDMOND [IE]) 24 September 2020 (2020-09-24)<br>* paragraph [0094] *<br>* paragraph [0111] – paragraph [0116] *<br>* paragraph [0124] *<br>* paragraph [0129] – paragraph [0130] *<br>* paragraph [0144] – paragraph [0149] *<br>* paragraph [0168] – paragraph [0183] *<br>* paragraph [0188] *<br>* paragraph [0300] – paragraph [0302] *<br>* figure 3 *<br>----- | 1-15 | INV.<br>A61B5/00<br>A61B5/024<br>A61B5/08<br>A61B5/145 |
| X | US 2020/135334 A1 (RAJASEKHAR VIJAYKUMAR [US] ET AL) 30 April 2020 (2020-04-30)<br>* paragraph [0100] – paragraph [0102] *<br>* paragraph [0139] *<br>* paragraph [0163] *<br>* paragraph [0180] *<br>* paragraph [0192] – paragraph [0221] *<br>----- | 1-15 | |
| | | | **TECHNICAL FIELDS SEARCHED (IPC)**<br><br>A61B<br>G01N |

The present search report has been drawn up for all claims

| Place of search | Date of completion of the search | Examiner |
|---|---|---|
| The Hague | 7 June 2023 | Gooding Arango, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 23 30 5058

This annex lists the patent family members relating to the patent documents cited in the above-mentioned European search report.
The members are as contained in the European Patent Office EDP file on
The European Patent Office is in no way liable for these particulars which are merely given for the purpose of information.

07-06-2023

| Patent document cited in search report | | Publication date | Patent family member(s) | | Publication date |
|---|---|---|---|---|---|
| US 2020297955 | A1 | 24-09-2020 | CN | 108135534 A | 08-06-2018 |
| | | | CN | 114588445 A | 07-06-2022 |
| | | | EP | 3340876 A2 | 04-07-2018 |
| | | | EP | 3838138 A2 | 23-06-2021 |
| | | | JP | 6987042 B2 | 22-12-2021 |
| | | | JP | 7284782 B2 | 31-05-2023 |
| | | | JP | 2018531055 A | 25-10-2018 |
| | | | JP | 2021180853 A | 25-11-2021 |
| | | | US | 2020297955 A1 | 24-09-2020 |
| | | | WO | 2017032873 A2 | 02-03-2017 |
| US 2020135334 | A1 | 30-04-2020 | US | 2020135334 A1 | 30-04-2020 |
| | | | WO | 2020087014 A1 | 30-04-2020 |

EPO FORM P0459

For more details about this annex : see Official Journal of the European Patent Office, No. 12/82

**EP 4 403 096 A1**

REFERENCES CITED IN THE DESCRIPTION

**Patent documents cited in the description**

- WO 2019158704 A
- WO 2021064212 A